# EUROPEAN PATENT SPECIFICATION

(11) **EP 4 452 352 B1**
(45) Date of publication and mention of the grant of the patent: **06.08.2025**
(21) Application number: 23739752.6
(22) Date of filing: 16.06.2023
(51) Int. Cl.: A61L 29/12

(54) **MEDICAL TUBE AND METHOD OF FORMATION**
MEDIZINISCHER SCHLAUCH UND VERFAHREN ZUR HERSTELLUNG
TUBE MÉDICAL ET PROCÉDÉ DE FORMATION

(30) Priority: 17.06.2022 US 202263353136 P
(43) Date of publication of application: 30.10.2024
(73) Proprietor: Tekni-Plex, Inc., Wayne, PA 19087 (US)
(72) Inventor: MCSHANE, Paul, Belfast, BT170QL (IE); KENNY, Ian, Belfast, BT170QL (IE); VAN LANDEGHEM, Robin, 9320 Erembodegem (BE)
(74) Representative: Page White Farrer
(86) International application number: PCT/US2023/025506
(87) International publication number: WO 2023/244771

(56) References cited:
- WO-A1-2013/109329
- US-A1- 2018 117 295
- US-A1- 2020 406 019
- US-B2- 10 646 704

## Description

### Field of the Invention

The present invention relates to polymeric tubing for delivering medical fluids to a patient and methods for formation of such tubing typically effected by a co-extrusion process wherein the tubing has multiple layers of the same or different polymeric materials each layer successively adhered to each other.

### Background

Tubing comprised of polymeric material is used in many industrial and commercial applications including in the medical field. Various FDA compliant plastics are used, depending upon properties desired and the intended applications. Where the tubing is used to transport fluids for in vivo treatment of human patients, selection of the polymeric materials can be a factor.

Three layer tubing using a polyethylene based polymer as an inner most layer that contacts medical fluid has been used where the innermost polyethylene layer is bonded to an outermost layer of polyurethane via a middle or intermediate layer of an adhesive as disclosed in U.S. Patent No. 10,646,704. While such a tubing structure has certain advantages, a polyethylene tubing layer that contacts a typical medical treatment aqueous fluid can negatively interact with certain commonly useful medical fluids such as aqueous compositions of insulin and other drugs.

Polyvinyl chloride (PVC) is one of the most widely used plastics. While structurally stable and easily formable into desired shapes, PVC is typically manufactured using plasticizers which can migrate out of the PVC matrix into bodily fluids and has other properties not ideally suited for medical treatment applications. Likewise, due to the inherent nature of plasticized PVC tubing, there arises the potential absorption of medicines and other components of aqueous fluids used in medical treatments into the sidewall of the PVC tube. Polyurethane is potentially a substitute for PVC. However, dual layer tubing comprised of polyurethane and polyethylene suffers from the inability of the two layers to remain adhered to each other under low to moderate stress, strain or mechanical manipulation conditions. U.S. Pat. No. 4,627,844 to Schmitt ("Schmitt") discloses a tri-layer tube which is embodied in a commercial product sold under the trademark "SUREPATH 151" by the Natvar Division of Tekni-Plex, Inc. As disclosed in Schmitt, an outer layer of PVC and an inner fluid-contact layer of low density polyethylene (LDPE) are co-extruded with an intermediate tie layer of ethylene vinyl acetate copolymer (EVA). However, while the inner polyethylene layer of Schmitt may be inert to or not interfere with the components of some medical fluids, polyethylene can negatively interact with components of other medical fluids such as many common fluid compositions that contain insulin.

### Summary of the Invention

As noted above, tubing with a polyethylene inner layer may negatively interact with medicinal components over an extended period.

In accordance with the invention, the selection of polypropylene as the inner layer polymeric material can prevent unwanted loss of ingredients/absorption medicinal components, protecting the integrity of the formulation over the normal course of use of the tubing delivering aqueous fluids, such as drug suspensions, chemotherapy drugs and insulin to or from a human subject.

Dual layer tubing comprising of an inner polypropylene layer and an outer polyurethane layer suffers from the inability of the two layers to remain adhered to each other.

This invention relates to an inner layer, an outer layer, a middle intermediate layer that binds the inner and outer layer together and prevents delamination of the multi-layered structure under moderate stress, strain or mechanical manipulation.

The selection of the appropriate middle layer polymeric material prevents delamination of the multilayered structure protecting the integrity of the tubing, tubing and fitment bonds and the formulation being delivered.

The polymeric materials of the middle layer is selected from one or more of ethylene vinyl acetate copolymers, ethylene acrylate copolymers, ethylene-acrylate maleic anhydride terpolymers, ethylene-acrylate glycidyl methacrylate terpolymers, and maleic anhydride grafted polypropylene.

Thermoplastic polyurethane as the outer layer is beneficial in medical tubing where PVC is not desired due to the presence of plasticizers. TPU provides secure bonding to fitments during post tube manufacturing operations and assembly of medical devices. Alternatively, the TPU outer layer may be substituted with a cyclic olefin copolymer, providing the desired performance characteristics at a lower cost.

In one embodiment of the invention, a tube (10) adapted for in vivo transport of an aqueous fluid to a subject comprising an inner layer (3), a middle layer (2) and an outer layer (1),
wherein the tube is formed by a co-extrusion process, the inner layer (3), middle layer (2) and outer layer (1) are concentric and the inner layer (3) and outer layer (1) are adhered to the middle layer (2) and thus adhered to each other, wherein
the inner layer (3) comprises a polypropylene,
the outer layer (1) comprises a thermoplastic polyurethane and
the middle layer (2) is selected from one or more of ethylene vinyl acetate copolymers, ethylene acrylate copolymers, ethylene-acrylate maleic anhydride terpolymers, ethylene-acrylate glycidyl methacrylate terpolymers, and maleic anhydride grafted polypropylene.

In one embodiment of the invention, the middle layer (2) comprises an ethylene ethyl acrylate copolymer or an ethylene methyl acrylate copolymer or an anhydride grafted ethylene methyl acrylate copolymer, a copolymer of two or more of said acrylates or a mixture of two or more of the foregoing.

In one embodiment of the invention, the middle layer (2) comprises ethylene vinyl acetate.

In one embodiment of the invention, the inner layer (3) comprises more than 90% by weight of a polypropylene homopolymer or a polypropylene copolymer, the outer layer (1) comprises more than 90% by weight of an aromatic or aliphatic polyether based polyurethane and the middle layer (2) comprises more than 90% by weight of the one or more of ethylene vinyl acetate copolymers, ethylene acrylate copolymers, ethylene-acrylate maleic anhydride terpolymers, ethylene-acrylate glycidyl methacrylate terpolymers, and maleic anhydride grafted polypropylene.

In one embodiment of the invention, the middle layer (2) comprises ethylene ethyl acrylate copolymer with at least 19.5 percent ethyl acrylate content by weight.

In one embodiment of the invention, the middle layer (2) comprises more than 90% by weight of the ethylene methyl acrylate copolymer.

In one embodiment of the invention, the middle layer (2) comprises ethylene vinyl acetate with at least 19.5 percent vinyl acetate content by weight.

In one embodiment of the invention, the inner layer (3) comprises more than 90% by weight of a polypropylene homopolymer or a polypropylene copolymer or a mixture of the homopolymer and copolymer, the outer layer (1) comprises more than 90% by weight of a polytetramethyleneglycol-based polyurethane.

In one embodiment of the invention, the thickness of the polyurethane outer layer is between 0.0254 and 0.635 mm (0.001 and 0.025 inches) the thickness of the inner polypropylene layer is between 0.0254 and 0.635 mm (0.001 and 0.025 inches) and the thickness of the middle layer is between 0.0254 and 0.635 mm (0.001 and 0.025 inches).

In one embodiment of the invention, the inner (3) and outer (1) layers do not visually delaminate from the middle layer (2) at a stress up to 20 MPa and a strain up to 400%.

In one embodiment of the invention, the tube (10) does not visually delaminate when submersed in water at 60° C for 72 hours.

In one embodiment of the invention, the tube (10) has a central axial fluid flow passage through which aqueous fluid is routed, the inner layer (3) having a radially inner wall surface that contacts the aqueous fluid the outer (1) and inner (3) layers resisting delamination from the middle layer (2) at a stress of up to 20 MPa and a strain of up to 400 %, and wherein the tube (10) does not visually delaminate after being submersed in water at 60° C for 72 hours.

In one embodiment of the invention, the inner (3) and outer (1) layers do not visually delaminate from the middle layer (2) at a stress of up to 20 MPa and a strain of up to 400%, and wherein the tube (10) does not visually delaminate after being submersed in water at 60° C for 72 hours.

In one embodiment of the invention, the outer layer (1) comprises more than 90% by weight of an aromatic polyether-based polyurethane, and
wherein the tube does not visually delaminate after being submersed in water at 60 °C for 72 hours.

In one embodiment of the invention, a method of forming a visually clear or transparent and manually flexible medical tube for in vivo transport of an aqueous fluid, the method of forming comprising:
selecting a first polymeric material that is an aromatic or aliphatic polyether based polyurethane material having a selected structural stability; and
selecting a second polymeric material that is a polypropylene material that is inert to aqueous fluids;
selecting a third polymeric material that is elastomeric in nature and has visual clarity and is selected from one or more of ethylene vinyl acetate copolymers, ethylene acrylate copolymers, ethylene-acrylate maleic anhydride terpolymers, ethylene-acrylate glycidyl methacrylate terpolymers, and maleic anhydride grafted polypropylene
co-extruding the selected first, second and third polymeric materials to form respectively adhered outer, inner and middle layers of the medical tube in a configuration such that the outer layer comprises at least 90% by weight of the first polymeric material, the inner layer comprises at least 90% weight of the second polymeric material and the middle layer comprises at least 90% by weight of the third polymeric material,
the materials being selected so as to maintain the integrity of the tube against delamination and maintain its visual clarity or transparency after being subjected to one or more of ethylene oxide and gamma irradiation sterilization,
wherein the medical tube has a central axial fluid flow passage defined by a radial inner wall surface of the inner layer through which aqueous fluid is transported, and wherein the tube does not visually delaminate after being submersed in water at 60 degrees C for 72 hours and subsequently mechanically flattened by manual squeezing of the tube from its normal round in cross-sectional condition to a flattened or oval cross-sectional shape or condition.

In one embodiment of the invention, a tube (10) adapted for in vivo transport of an aqueous fluid to a subject comprising an inner layer (3), a middle layer (2) and an outer layer (1),
wherein the tube is formed by a co-extrusion process, the inner layer (3), middle layer (2) and outer layer (1) are concentric and the inner layer (3) and outer layer (1) are adhered to the middle layer (2) and thus adhered to each other, wherein
the inner layer (3) comprises a polypropylene,
the outer layer (1) comprises a cyclic olefin copolymer and
the middle layer (2) is selected from one or more of ethylene vinyl acetate copolymers, ethylene acrylate copolymers, ethylene-acrylate maleic anhydride terpolymers, ethylene-acrylate glycidyl methacrylate terpolymers, and maleic anhydride grafted polypropylene.

In one embodiment of the invention, the inner layer (3) comprises more than 90% by weight of a polypropylene homopolymer or a polypropylene copolymer, the outer layer (1) comprises more than 90% by weight of a cyclic olefin coplymer and the middle layer (2) comprises more than 90% by weight of the one or more of ethylene vinyl acetate copolymers, ethylene acrylate copolymers, ethylene-acrylate maleic anhydride terpolymers, ethylene-acrylate glycidyl methacrylate terpolymers, and maleic anhydride grafted polypropylene.

In one embodiment of the invention, the inner (3) and outer (1) layers do not visually delaminate from the middle layer (2) at a stress of up to 20 MPa and a strain of up to 400%, and wherein the tube (10) does not visually delaminate after being submersed in water at 60° C for 72 hours.

In one embodiment of the invention, a method of forming a visually clear or transparent and manually flexible medical tube for in vivo transport of an aqueous fluid, the method of forming comprising:
selecting a first polymeric material that is an aromatic or aliphatic polyether based polyurethane material having a selected structural stability; and
selecting a second polymeric material that is a polypropylene material that is inert to aqueous fluids;
selecting a third polymeric material comprising an acrylate copolymer that is elastomeric in nature and has visual clarity and is selected from one or more of ethylene vinyl acetate copolymers, ethylene acrylate copolymers, ethylene-acrylate maleic anhydride terpolymers, ethylene-acrylate glycidyl methacrylate terpolymers, and maleic anhydride grafted polypropylene
co-extruding the selected first, second and third polymeric materials to form respectively adhered outer, inner and middle layers of the medical tube in a configuration such that the outer layer comprises at least 90% by weight of the first polymeric material, the inner layer comprises at least 90% weight of the second polymeric material and the middle layer comprises at least 90% by weight of the third polymeric material,
the materials being selected so as to maintain the integrity of the tube against delamination and maintain its visual clarity or transparency after being subjected to one or more of ethylene oxide and gamma irradiation sterilization,
wherein the medical tube has a central axial fluid flow passage defined by a radial inner wall surface of the inner layer through which aqueous fluid is transported, and wherein the tube does not visually delaminate after being submersed in water at 60 degrees C for 72 hours and subsequently mechanically flattened by manual squeezing of the tube from its normal round in cross-sectional condition to a flattened or oval cross-sectional shape or condition.

### Brief Description of the Drawings

The drawings depict one or more embodiments of the invention that are shown by way of examples of the invention wherein:

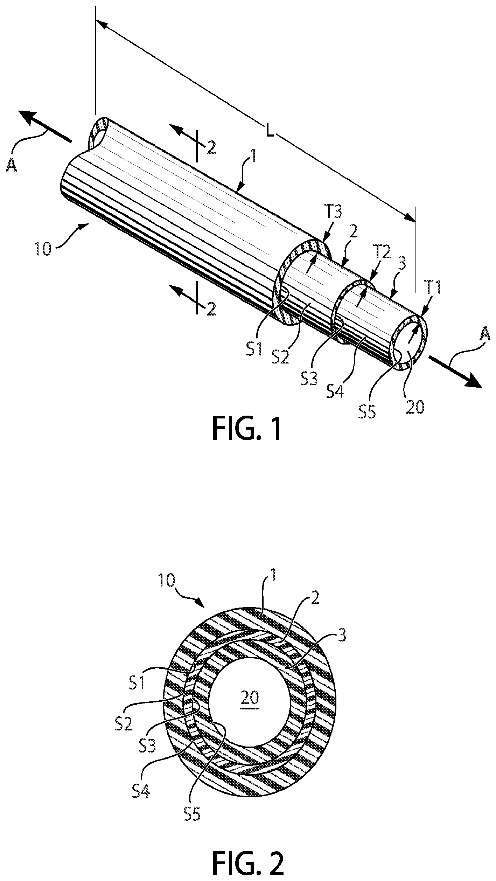

Fig. 1 is a schematic perspective view of a tri-layered tube showing the outer and middle layers broken away in order to better illustrate the construction and arrangement of the tubing;
Fig. 2 is a cross-sectional view taken along lines 2-2 of the tube 10 shown in Fig. 1.

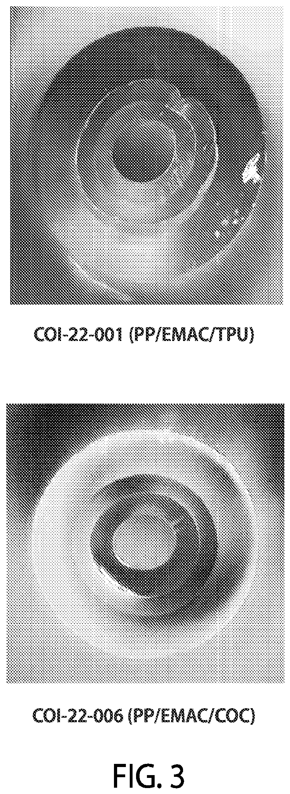

Fig. 3 are photos of a cross-section of tubes having inner, middle and outer layers, where the tubes were subjected to a tensile test using a Lloyd tensile testing instrument. The three layered tubes are subjected to a high tensile force pulling or stretching of the tubes to determine when and whether one layer pulls past another layer under the high tensional force applied and thus demonstrates the relative degree of adhesion of the middle layer to the inner and outer layers.

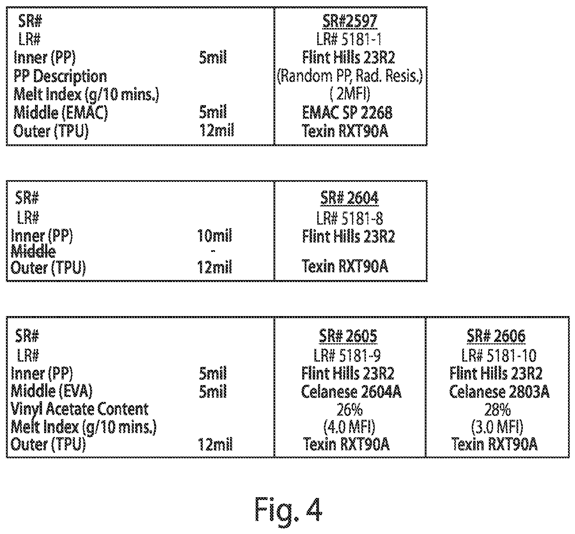

Fig. 4 is a matrix or chart illustrating layer thicknesses and compositions of one example of a 3M3L tube having an EMAC middle layer according to the invention (SR #2597), a comparison 2M2L example (SR# 2604), and two further examples of 3M3L tubes having an EVA middle layer according to the invention (SR#2605 and SR #2606).

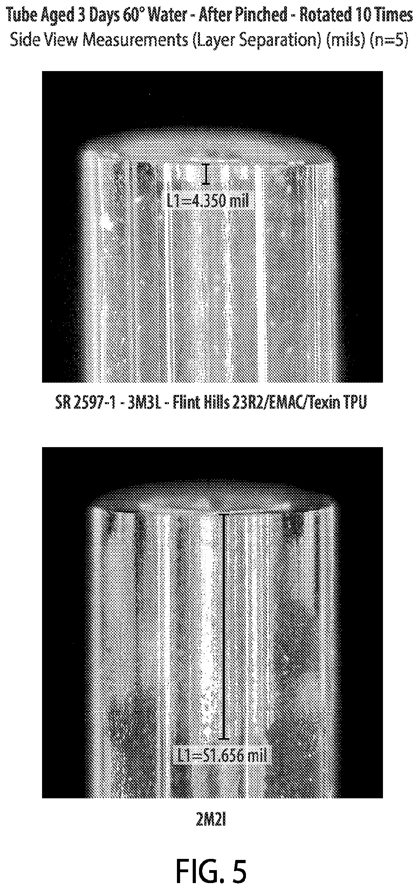

Fig. 5 are side view photos of two sample tubes, 3M3L SR #2597 according to the invention on the left, and comparison 2M2L on the right, showing layer separation visually and measured microscopically (L in mils (n=5)) for each tube aged 3 days in 60 degrees Centigrade distilled water, after being pinched and rotated 10 times, wherein the sample of the invention on the left had an L value of 4.350 mils, versus the comparison tube on the right had a much greater L value of 51.656 mils (significantly greater layer separation for the 2M2L tube( (1mil= 0.0254 mm).

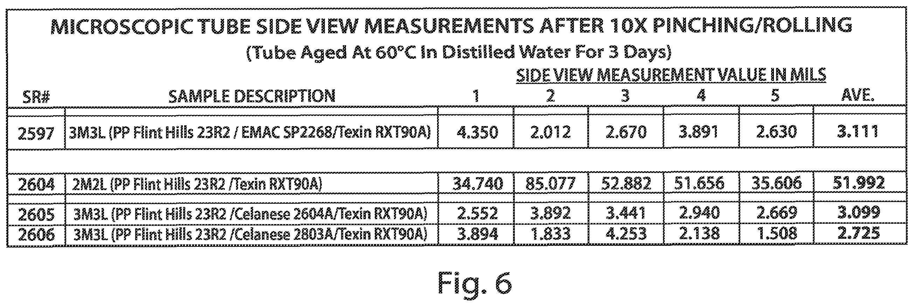

Fig. 6 is a matrix or chart illustrating side view microscopic measurements (L in mils) as in Fig. 4, listing 5 samples for each of SR #2597 (the invention, 3M3L with EMAC middle layer), SR #2604 (2M2L comparison), and Sr #2605 and SR #2606 (the invention, 3M3L with EVA middle layer), and an average L value for each group of 5 samples, again showing the 3M3L samples having much lower layer separation L values versus the 2M2L samples.

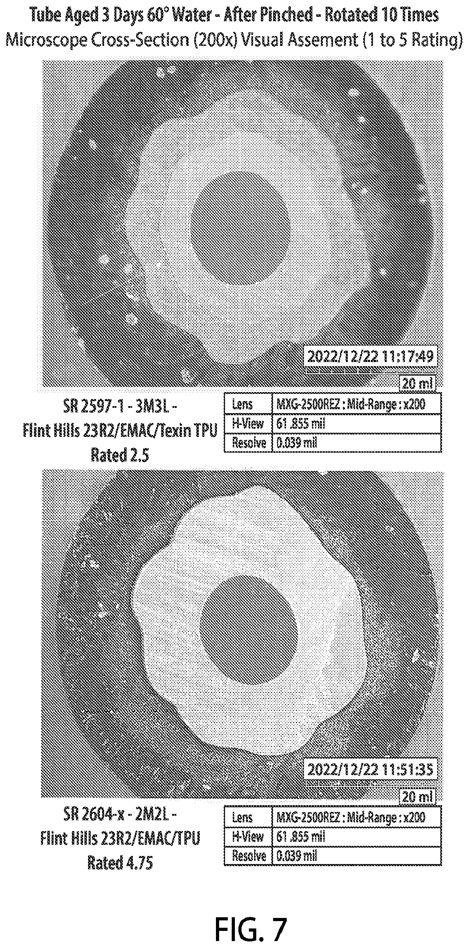

Fig. 7 are 200X microscopic cross sectional views of two sample tubes, 3M3L SR #2597 according to the invention on the left, and comparison 2M2L SR #2604 on the right, showing layer separation visually along with a visual assessment rating (1 to 5 rating) for each tube aged 3 days in 60 degrees Centigrade distilled water, after being pinched and rotated 10 times, wherein the sample of the invention on the left had an rating of 2.5, and the comparison tube on the right had a much greater rating value of 4.75 (a lower value is desired).

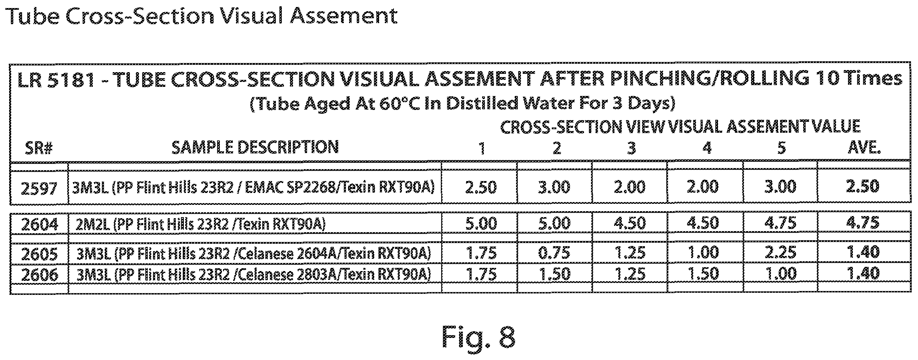

Fig. 8 is a matrix or chart illustrating cross sectional visual assessment ratings as in Fig. 6, listing 5 samples for each of SR #2597 (the invention, 3M3L with EMAC middle layer), SR #2604 (2M2L comparison), and Sr #2605 and SR #2606 (the invention 3M3L with EVA middle layer), and an average rating value for each group of 5 samples, again showing the 3M3L samples having much lower layer separation L values versus the 2M2L samples.

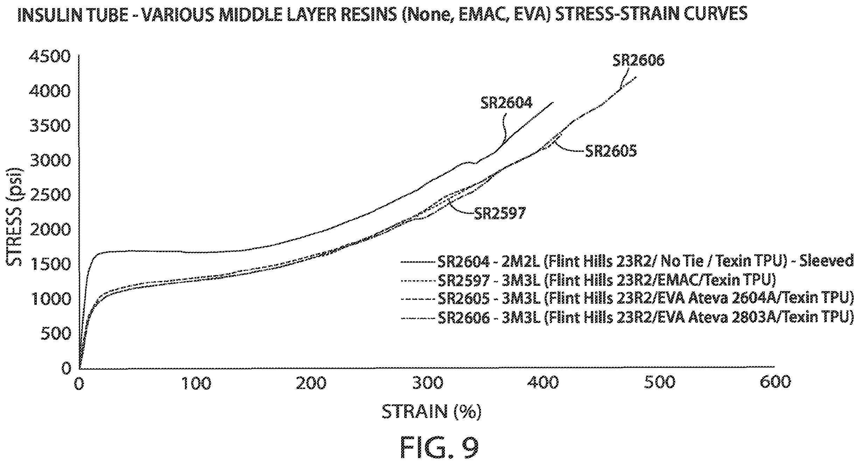

Fig. 9 is a graph showing tensile curves for one 2M2L (SR #2604) and various 3M3L tubes, each with different middle layers (EMAC SR #2597, EVA SR #2605 and EVA SR #2606), the tubes being configured for delivery of insulin, with stress (psi) on the vertical axis and strain (%) on the horizontal axis, wherein the 3M3L tubes all showed much lower layer separation (also referred to as sleeving) than the 2M2L sample.

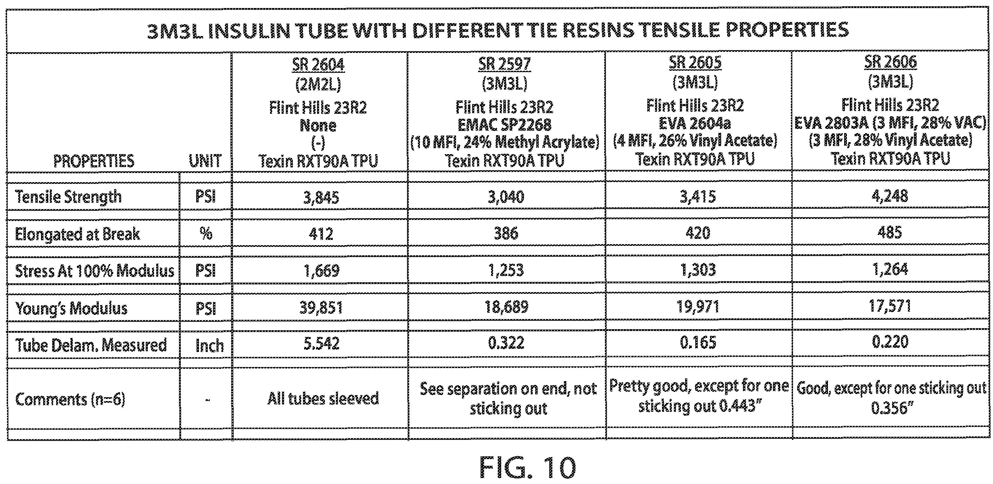

Fig. 10 is a matrix or chart illustrating various tensile properties for the various tubes shown in Fig. 8, namely 2M2L (SR #2604) and 3M3L tubes, with different middle layers in the 3M3L tubes (EMAC SR #2597, EVA SR #2605 and EVA SR #2606), wherein the 3M3L tubes all showed much better tensile properties and lower layer separation (also referred to as sleeving) than the 2M2L sample.

### Detailed Description

There is shown in FIG. 1 an embodiment of a co-extruded tri-layer tubing 10 according to the invention which comprises an outer layer 1 comprised of at least about 90% by weight of a polyurethane material, typically a polytetramethyleneglycol-based polyurethane one example of which is Lubrizol TPU Pellethane 2363-90AE. The outer layer can alternatively comprise a cyclic olefin copolymer material such as Topas E-140.

As shown in Fig. 1 the tube includes an inner fluid-contact layer 3 typically comprised of at least about 90% by weight of a polypropylene material such as Ineos R01C and a middle bonding layer 2 comprised of at least about 90% by weight of an ethylene ethyl acrylate copolymer, an ethylene methyl acrylate copolymer, an anhydride grafted ethylene methyl acrylate copolymer, a copolymer of two or more of said acrylates or a mixture of two or more of these acrylate based compounds or compositions. One example of a suitable ethylene ethyl acrylate copolymer is Dow Amplify EA 103 (Ethylene Ethyl Acrylate being about 19.5% by weight). Examples of suitable ethylene methyl acrylate copolymers are Westlake MA SP2268 (Ethylene Methyl Acrylate being about 24% by weight), Westlake MA SP2220 (Ethylene Methyl Acrylate being about 20% by weight). One example of a suitable anhydride grafted ethylene methyl acrylate copolymer is Westlake Tymax GA 7001 (Anhydride grafted Ethylene Methyl Acrylate). The middle layer can alternatively comprise an ethylene-vinyl acetate copolymer.

As shown in Fig. 1 the outer layer 1has a radially inner facing surface S1 that binds and adheres to a radially outer facing surface S2 of the middle layer 2. Similarly the inner layer 3 has a radially outer facing surface S4 that binds and adheres to the radially inner facing surface S3 of the middle layer 2. The middle layer 2 adheres to the outer 1 and inner 3 layers such that the layers 1 and 3 remain adhered to layer 2 and to each other when the tube 10 is subjected to a stress of up to about 20 MPa and a strain of up to about 400% as measured by pulling a length of tubing 10 of about 2 inches (linch= 2.54 cm) in axial length L along its axis A using a Lloyd LR5K Plus mechanical tester at a pull rate of about 12 inches/minute at ambient environmental conditions of about 22.2 degrees C (72 degrees F) and about 50% relative humidity, the break point of the tubing 10 being at about 17-22 MPa and about 300 to 450%. The layers 1, 2, 3 of such tubing 10 does not visually delaminate after being subjected to submersion in water at 60° C for 36 or 72 hours and subsequently mechanically flattened by manual squeezing of the tube from its normal round in cross-sectional condition to a flattened or oval shape cross-sectional shape or condition.

As shown in Figs. 1 and 2, the layers 1, 2, 3 are formed into structurally stable walls that surround and enclose a central hollow fluid passage 20 through which an aqueous solution is routed and flows in an axial A direction contacting the radially inner facing surface S5 of the inner layer 3. The middle layer 2 binds and holds the inner 3 and outer 1 layers together.

The inner layer 3 provides a radially inner fluid-contact surface S5, the thickness, of the inner layer 3 typically ranging in cross-sectional thickness T1 of between about 0.001 inches and about 0.025 inches. The middle layer 2 typically ranges in cross-sectional thickness T2 of between about 0.001 inches and about 0.025 inches. The outer layer 1 typically ranges in cross-sectional thickness T3 of between about 0.001 inches and about 0.025 inches.

The polypropylene material is preferably a homopolymer or a copolymer of propene and ethene or a mixture of the foregoing. A typical polypropylene material is Ineos R01C.

The polyurethane elastomer (TPU) is typically the reaction product of a polyol and isocyanate and usually includes a combination of hard and soft segment domains. An aromatic polyether-based TPU or an aliphatic polyether-based TPU can be used such as a polytetramethyleneglycol-based polyurethane. Preferred TPU's include the Pellethane 2363-80 AE series available from the Lubrizol Corporation such as Lubrizol TPU Pellethane 2363-90AE and BASF 1190A.

The respective thickness of each layer of tubing 10 can be controlled by the extrusion tooling utilized, such as the "Tri Die" extrusion apparatus manufactured by the Genca Division of General Cable Company, Clearwater, Fla. The extrusion apparatus is selected so as to provide a uniform thickness of the layers 1, 2, 3 along the substantial entirety of the axial length L of all three layers 1, 2, 3.

The polymeric materials of which the layers 1, 2, 3 are comprised are selected so as to be visually clear or transparent and manually flexible along and around the axis A of the tubing. The polymeric materials are also selected so as to maintain the integrity of the tubing 10 (namely delamination does not occur) and its transparency or clarity after being subjected to ethylene oxide (EtO) and gamma irradiation sterilization processes.

Fig. 3 are photos of cross-section of tubes having polypropylene inner, EMAC middle and one of TPU or COC on the outer layer. The photo on the left tube has an outer layer of polyurethane material, a middle layer of EMAC and an inner layer of polypropylene material. As can be seen from the cross-section of the tube that has been subjected to the high tensile pulling on the Lloyd instrument, the three layers remain substantially adhered to each other. Further as shown in Fig. 3, the tube on the right has the same polypropylene inner layer material and the same EMAC middle layer material but has an outer layer comprised of a cyclic olefin copolymer (COC) material. As shown, the three layers of this tube remain substantially adhered to each other similar to the tube on the left.

Further testing of various embodiments of the invention are described below and illustrated in Figs. 4-10. Various materials and layer constructions of samples according to the invention for three material three layer (3M3L) tubes were used as listed below, and a comparison sample two material two layer (2M2L) tube: Materials:
- PP Flint Hills 23R2
- EMAC EMAC SP2268
- EVA Celanese 2604A
   EVA Celanese 2803A
- TPU Texin RXT90A

### Samples:

SR2604 (2M2L) Comparison
   PP Flint Hills 23R2
   TPU Texin RXT90A
SR2597 (3M3L)
   PP Flint Hills 23R2
   EMAC SP2268
   TPU Texin RXT90A
SR2605 (3M3L)
   PP Flint Hills 23R2
   EVA Celanese 2604A
   TPU Texin RXT90A
SR2606 (3M3L)
   PP Flint Hills 23R2
   EVA Celanese 2803A
   TPU Texin RXT90A

Fig. 4 is a matrix or chart illustrating layer thicknesses and compositions of one example of a 3M3L tube having an EMAC middle layer according to the invention (SR #2597), a comparison 2M2L example (SR# 2604), and two further examples of 3M3L tubes having an EVA middle layer according to the invention (SR#2605 and SR #2606).

Fig. 5 are side view photos of two sample tubes, 3M3L SR #2597 according to the invention on the left, and comparison 2M2L on the right, showing layer separation visually and measured microscopically (L in mils (n=5)) for each tube aged 3 days in 60 degrees Centigrade distilled water, after being pinched and rotated 10 times, wherein the sample of the invention on the left had an L value of 4.350 mils, versus the comparison tube on the right had a much greater L value of 51.656 mils (significantly greater layer separation for the 2M2L tube).

Fig. 6 is a matrix or chart illustrating side view microscopic measurements (L in mils) as in Fig. 4, listing 5 samples for each of SR #2597 (the invention, 3M3L with EMAC middle layer), SR #2604 (2M2L comparison), and SR #2605 and SR #2606 (the invention, 3M3L with EVA middle layer), and an average L value for each group of 5 samples, again showing the 3M3L samples having much lower layer separation L values versus the 2M2L samples.

Fig. 7 are 200X microscopic cross sectional views of two sample tubes, 3M3L SR #2597 according to the invention on the left, and comparison 2M2L SR #2604 on the right, showing layer separation visually along with a visual assessment rating (1 to 5 rating) for each tube aged 3 days in 60 degrees Centigrade distilled water, after being pinched and rotated 10 times, wherein the sample of the invention on the left had an rating of 2.5, and the comparison tube on the right had a much greater rating value of 4.75 (a lower value is desired).

Fig. 8 is a matrix or chart illustrating cross sectional visual assessment ratings as in Fig. 6, listing 5 samples for each of SR #2597 (the invention, 3M3L with EMAC middle layer), SR #2604 (2M2L comparison), and Sr #2605 and SR #2606 (the invention 3M3L with EVA middle layer), and an average rating value for each group of 5 samples, again showing the 3M3L samples having much lower layer separation L values versus the 2M2L samples.

Fig. 9 is a graph showing tensile curves for one 2M2L (SR #2604) and various 3M3L tubes, each with different middle layers (EMAC SR #2597, EVA SR #2605 and EVA SR #2606), the tubes being configured for delivery of insulin, with stress (psi) on the vertical axis and strain (%) on the horizontal axis, wherein the 3M3L tubes all showed much lower layer separation (also referred to as sleeving) than the 2M2L sample.

Fig. 10 is a matrix or chart illustrating various tensile properties for the various tubes shown in Fig. 8, namely 2M2L (SR #2604) and 3M3L tubes, with different middle layers in the 3M3L tubes (EMAC SR #2597, EVA SR #2605 and EVA SR #2606), wherein the 3M3L tubes all showed much better tensile properties and lower layer separation (also referred to as sleeving) than the 2M2L sample.

In various embodiments, the middle layer typically serves as a barrier against migration of mobile moieties between or from the layers and the central flow passage, wherein the mobile moieties comprise monomers, short chained polymers, ions, water, small organic molecules, metals, plasticizers, and catalysts.

In various embodiments, the layers of polymeric materials are co-extruded together to form the tubing such that the outer and inner layers are adhered to the middle layer and thus adhered to each other. The tubing is formed with a central hollow channel, bore or passage that is radially surrounded and defined by the polymeric layers that act as the walls of the tubing.

With reference to Figs. 1, 2, preferably the outer layer 1 is between about 0.001" and about 0.025" in thickness, T3, the inner layer 3 is between about 0.001" and about 0.025" in thickness, T1, and the middle layer 2 is between about 0.001" and about 0.025" in thickness, T2. The layers 1, 2, 3 collectively form a tubular wall surrounding and defining a central fluid flow passage 20.

The polymeric materials are preferably "contaminant free" meaning that they do not contain more than insignificant amounts of potentially unwanted materials (typically less than about 0.5% and preferably less than about 0.2%, by weight) and/or prevent leaching or leaking of unwanted materials such as plasticizers, catalysts, monomers, metals, salts, ions or other substances that are potentially unwanted to a human being into an aqueous solution or medium with which one or the other of the three layers may come into contact during the normal course of use of the tubing in delivering aqueous fluid, such as insulin, chemotherapy drugs and other potentially unstable aqueous drug suspensions, to or from a human subject. In addition to acting as an adhesive between and adhering to the outer and inner layers, the middle layer prevents delamination of the outer and inner layers from the middle layers under conditions of relatively low to moderate stress or strain. In addition, the middle layer acts as a barrier to leaching or leaking of contaminants from the outer layer to or through the inner layer into the hollow central bore or passage of the tube.

The polypropylene material typically comprises one or more of a polypropylene homopolymer and a polypropylene copolymer.

Ethylene vinyl acetate (EVA), Ethylene ethyl acrylate copolymers (EEA), Ethylene methyl acrylate (EMA) copolymers and anhydride grated ethylene methyl acrylate (AEMA) copolymers are elastomeric in nature and have excellent visual clarity. In a typical 3M3L co-extrusion process, TPU, EVA, EEA or EMA or AEMA and PP are melt extruded through a die head to form a tubular shaped extrudate that is then cooled through conventional water baths or water vacuum tanks and which are either subsequently wound or cut into a particular length for use. The level of elasticity and softness of the EVA, EEA, EMA AEMA or copolymer thereof is controlled through the amount of vinyl acetate, ethyl acrylate or methyl acrylate comonomer utilized with ethylene in the copolymerization process. The resulting three layer tubes manufactured by such a co-extrusion process act in a monolithic manner in that they return to close to their original shape and dimensions after being strained or stretched in a tensile manner along the longitudinal axis of the tube at a stress of up to about 20 MPa and a strain of up to about 400 % and without any visual delamination between any of the layers after being submersed in water at about 60° C for about 36 hours.

The cyclic olefin copolymer is typically produced by:
chain copolymerization of a cyclic monomer with ethene, or
by ring-opening metathesis polymerization of a cyclic monomer followed by hydrogenation.

The cyclic olefin copolymer can be produced by: chain copolymerization with ethene of a cyclic monomer comprised of one or more of 8,9,10-trinorborn-2-ene and 1,2,3,4,4a,5,8,8a-octahydro-1,4.5,8-dimethanonaphthalene, or
by ring-opening metathesis polymerization of a cyclic monomer followed by hydrogenation.

## Claims

1. A tube (10) adapted for in vivo transport of an aqueous fluid to a subject comprising an inner layer (3), a middle layer (2) and an outer layer (1),
wherein the tube is formed by a co-extrusion process, the inner layer (3), middle layer (2) and outer layer (1) are concentric and the inner layer (3) and outer layer (1) are adhered to the middle layer (2) and thus adhered to each other, wherein
the inner layer (3) comprises a polypropylene,
the outer layer (1) comprises a thermoplastic polyurethane and
the middle layer (2) is selected from one or more of ethylene vinyl acetate copolymers, ethylene acrylate copolymers, ethylene-acrylate maleic anhydride terpolymers, ethylene-acrylate glycidyl methacrylate terpolymers, and maleic anhydride grafted polypropylene.

2. The tube of claim 1 wherein the middle layer (2) comprises an ethylene ethyl acrylate copolymer or an ethylene methyl acrylate copolymer or an anhydride grafted ethylene methyl acrylate copolymer, a copolymer of two or more of said acrylates or a mixture of two or more of the foregoing.

3. The tube of claim 1 wherein the middle layer (2) comprises ethylene vinyl acetate.

4. The tube (10) of claim 1 wherein the inner layer (3) comprises more than 90% by weight of a polypropylene homopolymer or a polypropylene copolymer, the outer layer (1) comprises more than 90% by weight of an aromatic or aliphatic polyether based polyurethane and the middle layer (2) comprises more than 90% by weight of the one or more of ethylene vinyl acetate copolymers, ethylene acrylate copolymers, ethylene-acrylate maleic anhydride terpolymers, ethylene-acrylate glycidyl methacrylate terpolymers, and maleic anhydride grafted polypropylene.

5. The tube of claim 4 wherein the middle layer (2) comprises ethylene ethyl acrylate copolymer with at least 19.5 percent ethyl acrylate content by weight.

6. The tube of claim 4 wherein the middle layer (2) comprises more than 90% by weight of the ethylene methyl acrylate copolymer.

7. The tube of claim 4 wherein the middle layer (2) comprises ethylene vinyl acetate with at least 19.5 percent vinyl acetate content by weight.

8. The tube of claim 1 wherein the inner layer (3) comprises more than 90% by weight of a polypropylene homopolymer or a polypropylene copolymer or a mixture of the homopolymer and copolymer, the outer layer (1) comprises more than 90% by weight of a polytetramethyleneglycol-based polyurethane.

9. The tube of claim 1 wherein the thickness of the polyurethane outer layer is between 0.0254 and 0.635 mm (0.001 and 0.025 inches) the thickness of the inner polypropylene layer is between 0.0254 and 0.635 mm (0.001 and 0.025 inches) and the thickness of the middle layer is between 0.0254 and 0.635 mm (0.001 and 0.025 inches).

10. The tube of claim 1 wherein the inner (3) and outer (1) layers do not visually delaminate from the middle layer (2) at a stress up to 20 MPa and a strain up to 400%.

11. The tube of claim 1 wherein the tube (10) does not visually delaminate when submersed in water at 60° C for 72 hours.

12. The tube of claim 1 wherein the tube (10) has a central axial fluid flow passage through which aqueous fluid is routed, the inner layer (3) having a radially inner wall surface that contacts the aqueous fluid the outer (1) and inner (3) layers resisting delamination from the middle layer (2) at a stress of up to 20 MPa and a strain of up to 400 %, and wherein the tube (10) does not visually delaminate after being submersed in water at 60° C for 72 hours.

13. The tube of claim 1 wherein the inner (3) and outer (1) layers do not visually delaminate from the middle layer (2) at a stress of up to 20 MPa and a strain of up to 400%, and wherein the tube (10) does not visually delaminate after being submersed in water at 60° C for 72 hours.

14. The tube of claim 1 wherein the outer layer (1) comprises more than 90% by weight of an aromatic polyether-based polyurethane, and
wherein the tube does not visually delaminate after being submersed in water at 60 °C for 72 hours.

15. A method of forming a visually clear or transparent and manually flexible medical tube for in vivo transport of an aqueous fluid, the method of forming comprising:
selecting a first polymeric material that is an aromatic or aliphatic polyether based polyurethane material having a selected structural stability; and
selecting a second polymeric material that is a polypropylene material that is inert to aqueous fluids;
selecting a third polymeric material that is elastomeric in nature and has visual clarity and is selected from one or more of ethylene vinyl acetate copolymers, ethylene acrylate copolymers, ethylene-acrylate maleic anhydride terpolymers, ethylene-acrylate glycidyl methacrylate terpolymers, and maleic anhydride grafted polypropylene; and
co-extruding the selected first, second and third polymeric materials to form respectively adhered outer, inner and middle layers of the medical tube in a configuration such that the outer layer comprises at least 90% by weight of the first polymeric material, the inner layer comprises at least 90% weight of the second polymeric material and the middle layer comprises at least 90% by weight of the third polymeric material,
the materials being selected so as to maintain the integrity of the tube against delamination and maintain its visual clarity or transparency after being subjected to one or more of ethylene oxide and gamma irradiation sterilization,
wherein the medical tube has a central axial fluid flow passage defined by a radial inner wall surface of the inner layer through which aqueous fluid is transported, and wherein the tube does not visually delaminate after being submersed in water at 60 degrees C for 72 hours and subsequently mechanically flattened by manual squeezing of the tube from its normal round in cross-sectional condition to a flattened or oval cross-sectional shape or condition.

16. A tube (10) adapted for in vivo transport of an aqueous fluid to a subject comprising an inner layer (3), a middle layer (2) and an outer layer (1),
wherein the tube is formed by a co-extrusion process, the inner layer (3), middle layer (2) and outer layer (1) are concentric and the inner layer (3) and outer layer (1) are adhered to the middle layer (2) and thus adhered to each other, wherein
the inner layer (3) comprises a polypropylene,
the outer layer (1) comprises a cyclic olefin coplymer and
the middle layer (2) is selected from one or more of ethylene vinyl acetate copolymers, ethylene acrylate copolymers, ethylene-acrylate maleic anhydride terpolymers, ethylene-acrylate glycidyl methacrylate terpolymers, and maleic anhydride grafted polypropylene.

17. The tube (10) of claim 16 wherein the inner layer (3) comprises more than 90% by weight of a polypropylene homopolymer or a polypropylene copolymer, the outer layer (1) comprises more than 90% by weight of a cyclic olefin copolymer and the middle layer (2) comprises more than 90% by weight of the one or more of ethylene vinyl acetate copolymers, ethylene acrylate copolymers, ethylene-acrylate maleic anhydride terpolymers, ethylene-acrylate glycidyl methacrylate terpolymers, and maleic anhydride grafted polypropylene.

18. The tube of claim 16 wherein the inner (3) and outer (1) layers do not visually delaminate from the middle layer (2) at a stress of up to 20 MPa and a strain of up to 400%, and wherein the tube (10) does not visually delaminate after being submersed in water at 60° C for 72 hours.

## Patentansprüche

1. Eine Röhre (10), geeignet für den in-vivo-Transport einer wässrigen Flüssigkeit zu einem Subjekt, umfassend eine innere Schicht (3), eine mittlere Schicht (2) und eine äußere Schicht (1),
wobei die Röhre durch ein Koextrusionsverfahren gebildet ist, die innere Schicht (3), die mittlere Schicht (2) und die äußere Schicht (1) konzentrisch sind und die innere Schicht (3) sowie die äußere Schicht (1) an der mittleren Schicht (2) haften und somit miteinander verbunden sind, wobei
die innere Schicht (3) Polypropylen umfasst,
die äußere Schicht (1) thermoplastisches Polyurethan umfasst und
die mittlere Schicht (2) ausgewählt ist aus einem oder mehreren der folgenden: Ethylen-Vinylacetat-Copolymere, Ethylen-Acrylat-Copolymere, Ethylen-Acrylat-Maleinsäureanhydrid-Terpolymere, Ethylen-Acrylat-Glycidylmethacrylat-Terpolymere und mit Maleinsäureanhydrid gepfropftes Polypropylen.

2. Die Röhre nach Anspruch 1, wobei die mittlere Schicht (2) ein Ethylen-Ethylacrylat-Copolymer oder ein Ethylen-Methylacrylat-Copolymer oder ein mit Anhydrid gepfropftes Ethylen-Methylacrylat-Copolymer, ein Copolymer aus zwei oder mehr der genannten Acrylate oder eine Mischung aus zwei oder mehr der vorgenannten umfasst.

3. Die Röhre nach Anspruch 1, wobei die mittlere Schicht (2) Ethylen-Vinylacetat umfasst.

4. Die Röhre (10) nach Anspruch 1, wobei die innere Schicht (3) zu mehr als 90 Gew.-% aus einem Polypropylen-Homopolymer oder einem Polypropylen-Copolymer besteht, die äußere Schicht (1) zu mehr als 90 Gew.-% aus einem aromatischen oder aliphatischen Polyether-basierten Polyurethan besteht und die mittlere Schicht (2) zu mehr als 90 Gew.-% aus einem oder mehreren der folgenden besteht: Ethylen-Vinylacetat-Copolymere, Ethylen-Acrylat-Copolymere, Ethylen-Acrylat-Maleinsäureanhydrid-Terpolymere, Ethylen-Acrylat-Glycidylmethacrylat-Terpolymere und mit Maleinsäureanhydrid gepfropftes Polypropylen.

5. Die Röhre nach Anspruch 4, wobei die mittlere Schicht (2) ein Ethylen-Ethylacrylat-Copolymer mit einem Ethylacrylatgehalt von mindestens 19,5 Gew.-% umfasst.

6. Die Röhre nach Anspruch 4, wobei die mittlere Schicht (2) zu mehr als 90 Gew.-% aus dem Ethylen-Methylacrylat-Copolymer besteht.

7. Die Röhre nach Anspruch 4, wobei die mittlere Schicht (2) Ethylen-Vinylacetat mit einem Vinylacetatgehalt von mindestens 19,5 Gew.-% umfasst.

8. Die Röhre nach Anspruch 1, wobei die innere Schicht (3) zu mehr als 90 Gew.-% aus einem Polypropylen-Homopolymer oder einem Polypropylen-Copolymer oder einer Mischung aus Homopolymer und Copolymer besteht, die äußere Schicht (1) zu mehr als 90 Gew.-% aus einem Polyurethan auf Basis von Polytetramethylenglykol besteht.

9. Die Röhre nach Anspruch 1, wobei die Dicke der äußeren Polyurethan-Schicht zwischen 0,0254 und 0,635 mm (0,001 und 0,025 Zoll), die Dicke der inneren PolypropylenSchicht zwischen 0,0254 und 0,635 mm (0,001 und 0,025 Zoll) und die Dicke der mittleren Schicht zwischen 0,0254 und 0,635 mm (0,001 und 0,025 Zoll) liegt.

10. Die Röhre nach Anspruch 1, wobei sich die inneren (3) und äußeren (1) Schichten bei einer Spannung von bis zu 20 MPa und einer Dehnung von bis zu 400 % visuell nicht von der mittleren Schicht (2) delaminieren.

11. Die Röhre nach Anspruch 1, wobei sich die Röhre (10) bei Eintauchen in Wasser bei 60 °C über 72 Stunden visuell nicht delaminiert.

12. Die Röhre nach Anspruch 1, wobei die Röhre (10) einen zentralen axialen Flüssigkeitsdurchflusskanal aufweist, durch den wässrige Flüssigkeit geleitet wird, wobei die innere Schicht (3) eine radial innere Wandfläche aufweist, die mit der wässrigen Flüssigkeit in Kontakt steht, die äußere (1) und innere (3) Schicht einer Delamination von der mittleren Schicht (2) bei einer Spannung von bis zu 20 MPa und einer Dehnung von bis zu 400 % widerstehen, und wobei sich die Röhre (10) nach Eintauchen in Wasser bei 60 °C über 72 Stunden visuell nicht delaminiert.

13. Die Röhre nach Anspruch 1, wobei sich die inneren (3) und äußeren (1) Schichten bei einer Spannung von bis zu 20 MPa und einer Dehnung von bis zu 400 % visuell nicht von der mittleren Schicht (2) delaminieren und wobei sich die Röhre (10) nach Eintauchen in Wasser bei 60 °C über 72 Stunden visuell nicht delaminiert.

14. Die Röhre nach Anspruch 1, wobei die äußere Schicht (1) zu mehr als 90 Gew.-% aus einem aromatischen Polyether-basierten Polyurethan besteht und wobei sich die Röhre nach Eintauchen in Wasser bei 60 °C über 72 Stunden visuell nicht delaminiert.

15. Ein Verfahren zur Herstellung einer visuell klaren oder transparenten und manuell flexiblen medizinischen Röhre für den in-vivo-Transport einer wässrigen Flüssigkeit, wobei das Verfahren umfasst:
Auswählen eines ersten polymeren Materials, das ein aromatisches oder aliphatisches Polyether-basiertes Polyurethan-Material mit ausgewählter struktureller Stabilität ist; und
Auswählen eines zweiten polymeren Materials, das ein gegenüber wässrigen Flüssigkeiten inertes Polypropylen-Material ist;
Auswählen eines dritten polymeren Materials, das elastomerartig ist, visuelle Klarheit aufweist und ausgewählt ist aus einem oder mehreren der folgenden: Ethylen-Vinylacetat-Copolymere, Ethylen-Acrylat-Copolymere, Ethylen-Acrylat-Maleinsäureanhydrid-Terpolymere, Ethylen-Acrylat-Glycidylmethacrylat-Terpolymere und mit Maleinsäureanhydrid gepfropftes Polypropylen; und
Koextrudieren der ausgewählten ersten, zweiten und dritten polymeren Materialien zur Bildung jeweils haftender äußerer, innerer und mittlerer Schichten der medizinischen Röhre in einer Konfiguration, bei der die äußere Schicht mindestens 90 Gew.-% des ersten polymeren Materials, die innere Schicht mindestens 90 Gew.-% des zweiten polymeren Materials und die mittlere Schicht mindestens 90 Gew.-% des dritten polymeren Materials umfasst,
wobei die Materialien so ausgewählt sind, dass die Integrität der Röhre gegen Delamination erhalten bleibt und ihre visuelle Klarheit oder Transparenz nach einer oder mehreren Sterilisierungen mit Ethylenoxid oder Gammastrahlung erhalten bleibt, wobei die medizinische Röhre einen zentralen axialen Flüssigkeitsdurchflusskanal aufweist, der durch eine radial innere Wandfläche der inneren Schicht definiert ist, durch die wässrige Flüssigkeit transportiert wird, und wobei sich die Röhre nach Eintauchen in Wasser bei 60 °C über 72 Stunden und anschließendem mechanischen Abflachen durch manuelles Zusammendrücken der Röhre von ihrer normalen runden Querschnittsform in eine abgeflachte oder ovale Querschnittsform visuell nicht delaminiert.

16. Eine Röhre (10), geeignet für den in-vivo-Transport einer wässrigen Flüssigkeit zu einem Subjekt, umfassend eine innere Schicht (3), eine mittlere Schicht (2) und eine äußere Schicht (1),
wobei die Röhre durch ein Koextrusionsverfahren gebildet ist, die innere Schicht (3), die mittlere Schicht (2) und die äußere Schicht (1) konzentrisch sind und die innere Schicht (3) sowie die äußere Schicht (1) an der mittleren Schicht (2) haften und somit miteinander verbunden sind, wobei
die innere Schicht (3) Polypropylen umfasst,
die äußere Schicht (1) ein cyclisches Olefin-Copolymer umfasst und
die mittlere Schicht (2) ausgewählt ist aus einem oder mehreren der folgenden: Ethylen-Vinylacetat-Copolymere, Ethylen-Acrylat-Copolymere, Ethylen-Acrylat-Maleinsäureanhydrid-Terpolymere, Ethylen-Acrylat-Glycidylmethacrylat-Terpolymere und mit Maleinsäureanhydrid gepfropftes Polypropylen.

17. Die Röhre (10) nach Anspruch 16, wobei die innere Schicht (3) zu mehr als 90 Gew.-% aus einem Polypropylen-Homopolymer oder einem Polypropylen-Copolymer besteht, die äußere Schicht (1) zu mehr als 90 Gew.-% aus einem cyclischen Olefin-Copolymer besteht und die mittlere Schicht (2) zu mehr als 90 Gew.-% aus einem oder mehreren der folgenden besteht: Ethylen-Vinylacetat-Copolymere, Ethylen-Acrylat-Copolymere, Ethylen-Acrylat-Maleinsäureanhydrid-Terpolymere, Ethylen-Acrylat-Glycidylmethacrylat-Terpolymere und mit Maleinsäureanhydrid gepfropftes Polypropylen.

18. Die Röhre nach Anspruch 16, wobei sich die inneren (3) und äußeren (1) Schichten bei einer Spannung von bis zu 20 MPa und einer Dehnung von bis zu 400 % visuell nicht von der mittleren Schicht (2) delaminieren und wobei sich die Röhre (10) nach Eintauchen in Wasser bei 60 °C über 72 Stunden visuell nicht delaminiert.

## Revendications

1. Tube (10) adapté au transport in vivo d'un fluide aqueux vers un sujet, comprenant une couche interne (3), une couche intermédiaire (2) et une couche externe (1),
dans lequel le tube est formé par un procédé de coextrusion, la couche interne (3), la couche intermédiaire (2) et la couche externe (1) sont concentriques, et la couche interne (3) et la couche externe (1) sont adhérées à la couche intermédiaire (2) et ainsi adhérées l'une à l'autre,
dans lequel :
la couche interne (3) comprend un polypropylène,
la couche externe (1) comprend un polyuréthane thermoplastique, et
la couche intermédiaire (2) est sélectionnée parmi l'un ou plusieurs de copolymères d'éthylène-acétate de vinyle, de copolymères d'éthylène-acrylate, de terpolymères d'éthylène-acrylate-anhydride maléique, de terpolymères d'éthylène-acrylate-méthacrylate de glycidyle et d'un polypropylène greffé à l'anhydride maléique.

2. Tube selon la revendication 1, dans lequel la couche intermédiaire (2) comprend un copolymère d'éthylène-acrylate d'éthyle ou un copolymère d'éthylène-acrylate de méthyle ou un copolymère d'éthylène-acrylate de méthyle greffé à l'anhydride, un copolymère de deux ou plusieurs desdits acrylates ou un mélange de deux ou plusieurs de ce qui précède.

3. Tube selon la revendication 1, dans lequel la couche intermédiaire (2) comprend de l'éthylène-acétate de vinyle.

4. Tube (10) selon la revendication 1, dans lequel la couche interne (3) comprend plus de 90 % en poids d'un homopolymère de polypropylène ou d'un copolymère de polypropylène, la couche externe (1) comprend plus de 90 % en poids d'un polyuréthane à base de polyéther aromatique ou aliphatique, et la couche intermédiaire (2) comprend plus de 90 % en poids desdits l'un ou plusieurs de copolymères d'éthylène-acétate de vinyle, de copolymères d'éthylène-acrylate, de terpolymères d'éthylène-acrylate-anhydride maléique, de terpolymères d'éthylène-acrylate-méthacrylate de glycidyle et d'un polypropylène greffé à l'anhydride maléique.

5. Tube selon la revendication 4, dans lequel la couche intermédiaire (2) comprend un copolymère d'éthylène-acrylate d'éthyle avec une teneur en acrylate d'éthyle d'au moins 19,5 % en poids.

6. Tube selon la revendication 4, dans lequel la couche intermédiaire (2) comprend plus de 90 % en poids du copolymère d'éthylène-acrylate de méthyle.

7. Tube selon la revendication 4, dans lequel la couche intermédiaire (2) comprend de l'éthylène-acétate de vinyle avec une teneur en acétate de vinyle d'au moins 19,5 % en poids.

8. Tube selon la revendication 1, dans lequel la couche interne (3) comprend plus de 90 % en poids d'un homopolymère de polypropylène, d'un copolymère de polypropylène ou d'un mélange de l'homopolymère et du copolymère, la couche externe (1) comprenant plus de 90 % en poids d'un polyuréthane à base de polytétraméthylèneglycol.

9. Tube selon la revendication 1, dans lequel l'épaisseur de la couche externe en polyuréthane est comprise entre 0,0254 et 0,635 mm (0,001 et 0,025 pouces), l'épaisseur de la couche interne en polypropylène est comprise entre 0,0254 et 0,635 mm (0,001 et 0,025 pouces), et l'épaisseur de la couche intermédiaire est comprise entre 0,0254 et 0,635 mm (0,001 et 0,025 pouces).

10. Tube selon la revendication 1, dans lequel les couches interne (3) et externe (1) ne se délaminent pas visuellement de la couche intermédiaire (2) sous une contrainte allant jusqu'à 20 MPa et une déformation allant jusqu'à 400 %.

11. Tube selon la revendication 1, dans lequel le tube (10) ne se délamine pas visuellement lorsqu'il est immergé dans de l'eau à 60 °C pendant 72 heures.

12. Tube selon la revendication 1, dans lequel le tube (10) présente un passage axial central d'écoulement de fluide à travers lequel un fluide aqueux est acheminé, la couche interne (3) ayant une surface de paroi interne orientée radialement qui est en contact avec le fluide aqueux, les couches externe (1) et interne (3) résistant à la délamination de la couche intermédiaire (2) sous une contrainte allant jusqu'à 20 MPa et une déformation allant jusqu'à 400 %, et dans lequel le tube (10) ne se délamine pas visuellement après immersion dans de l'eau à 60 °C pendant 72 heures.

13. Tube selon la revendication 1, dans lequel les couches interne (3) et externe (1) ne se délaminent pas visuellement de la couche intermédiaire (2) sous une contrainte allant jusqu'à 20 MPa et une déformation allant jusqu'à 400 %, et dans lequel le tube (10) ne se délamine pas visuellement après immersion dans de l'eau à 60 °C pendant 72 heures.

14. Tube selon la revendication 1, dans lequel la couche externe (1) comprend plus de 90 % en poids d'un polyuréthane à base de polyéther aromatique, et dans lequel le tube ne se délamine pas visuellement après immersion dans de l'eau à 60 °C pendant 72 heures.

15. Procédé de fabrication d'un tube médical visuellement clair ou transparent et flexible manuellement pour le transport in vivo d'un fluide aqueux, le procédé de fabrication consistant à
sélectionner un premier matériau polymère qui est un matériau de polyuréthane à base de polyéther aromatique ou aliphatique ayant une stabilité structurelle sélectionnée ;
sélectionner un deuxième matériau polymère qui est un matériau de polypropylène inerte vis-à-vis des fluides aqueux ;
sélectionner un troisième matériau polymère qui est de nature élastomère, a une clarté visuelle, et est sélectionné parmi l'un ou plusieurs de copolymères d'éthylène-acétate de vinyle, de copolymères d'éthylène-acrylate, de terpolymères d'éthylène-acrylate-anhydride maléique, de terpolymères d'éthylène-acrylate-méthacrylate de glycidyle et d'un polypropylène greffé à l'anhydride maléique ;
coextruder les premier, deuxième et troisième matériaux polymères sélectionnés afin de former respectivement des couches externe, interne et intermédiaire adhérées du tube médical dans une configuration telle que la couche externe comprend au moins 90 % en poids du premier matériau polymère, la couche interne comprend au moins 90 % en poids du deuxième matériau polymère, et la couche intermédiaire comprend au moins 90 % en poids du troisième matériau polymère,
les matériaux étant sélectionnés de manière à maintenir l'intégrité du tube contre la délamination et à conserver sa clarté ou transparence visuelle après avoir été soumis à une ou plusieurs stérilisations par oxyde d'éthylène et irradiation gamma,
dans lequel le tube médical présente un passage axial central d'écoulement de fluide défini par une surface de paroi radiale interne de la couche interne à travers laquelle le fluide aqueux est transporté, et dans lequel le tube ne se délamine pas visuellement après immersion dans de l'eau à 60 °C pendant 72 heures et après avoir été mécaniquement aplati par compression manuelle du tube depuis sa forme normale circulaire en coupe transversale vers une forme ou condition aplatie ou ovale en coupe transversale.

16. Tube (10) adapté au transport in vivo d'un fluide aqueux vers un sujet, comprenant une couche interne (3), une couche intermédiaire (2) et une couche externe (1),
dans lequel le tube étant formé par un procédé de coextrusion, la couche interne (3), la couche intermédiaire (2) et la couche externe (1) sont concentriques, et la couche interne (3) et la couche externe (1) sont adhérées à la couche intermédiaire (2) et ainsi adhérées l'une à l'autre,
dans lequel :
la couche interne (3) comprend un polypropylène,
la couche externe (1) comprenant un copolymère d'oléfine cyclique, et
la couche intermédiaire (2) est sélectionnée parmi l'un ou plusieurs de copolymères d'éthylène-acétate de vinyle, de copolymères d'éthylène-acrylate, de terpolymères d'éthylène-acrylate-anhydride maléique, de terpolymères d'éthylène-acrylate-méthacrylate de glycidyle et d'un polypropylène greffé à l'anhydride maléique.

17. Tube (10) selon la revendication 16, dans lequel la couche interne (3) comprend plus de 90 % en poids d'un homopolymère de polypropylène ou d'un copolymère de polypropylène, la couche externe (1) comprend plus de 90 % en poids d'un copolymère d'oléfine cyclique, et la couche intermédiaire (2) comprend plus de 90 % en poids de l'un ou plusieurs de copolymères d'éthylène-acétate de vinyle, de copolymères d'éthylène-acrylate, de terpolymères d'éthylène-acrylate-anhydride maléique, de terpolymères d'éthylène-acrylate-méthacrylate de glycidyle et d'un polypropylène greffé à l'anhydride maléique.

18. Tube selon la revendication 16, dans lequel les couches interne (3) et externe (1) ne se délaminent pas visuellement de la couche intermédiaire (2) sous une contrainte allant jusqu'à 20 MPa et une déformation allant jusqu'à 400 %, et dans lequel le tube (10) ne se délamine pas visuellement après immersion dans de l'eau à 60 °C pendant 72 heures.
